(19) Europäisches Patentamt
European Patent Office
Office européen des brevets

(11) EP 2 779 189 A2

(12) # EUROPEAN PATENT APPLICATION

(43) Date of publication:
**17.09.2014 Bulletin 2014/38**

(51) Int Cl.:
***H01G 9/20*** *(2006.01)*

(21) Application number: **14152934.7**

(22) Date of filing: **28.01.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.03.2013 KR 20130025744**

(71) Applicant: **Samsung SDI Co., Ltd.**
**Yongin-si, Gyeonggi-do (KR)**

(72) Inventors:
- **Cha, Si-Young**
**Gyeonggi-do (KR)**
- **Lee, Ji-Won**
**Gyeonggi-do (KR)**
- **Kang, Moon-Sung**
**Gyeonggi-do (KR)**
- **Lee, Maeng-Eun**
**Gyeonggi-do (KR)**
- **Kang, Yong-Soo**
**Seoul (KR)**
- **Cho, Woo-Hyung**
**Seoul (KR)**

(74) Representative: **Russell, Tim et al**
**Venner Shipley LLP**
**200 Aldersgate**
**London EC1A 4HD (GB)**

(54) **Electrolyte for Dye-Sensitized Solar Cell and Dye-Sensitized Solar Cell Using the Same**

(57) In one aspect, an electrolyte for a dye-sensitized solar cell comprising a redox couple including a compound represented by Formula 1, and a dye-sensitized solar cell including the same are provided.

**Formula 1**

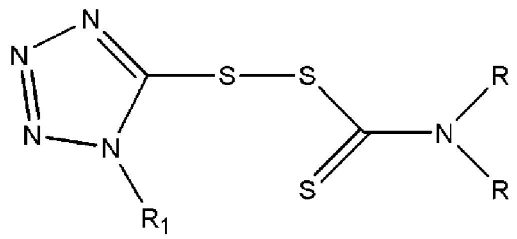

wherein, **R** and **$R_1$** each independently are selected from a substituted or unsubstituted $C_1$-$C_{20}$ alkyl group, a substituted or unsubstituted $C_6$-$C_{20}$ aryl group, a substituted or unsubstituted $C_1$-$C_{20}$ heteroaryl group, a substituted or unsubstituted $C_4$-$C_{20}$ carbocyclic group, and a substituted or unsubstituted $C_1$-$C_{20}$ heterocyclic group

EP 2 779 189 A2

FIG. 1

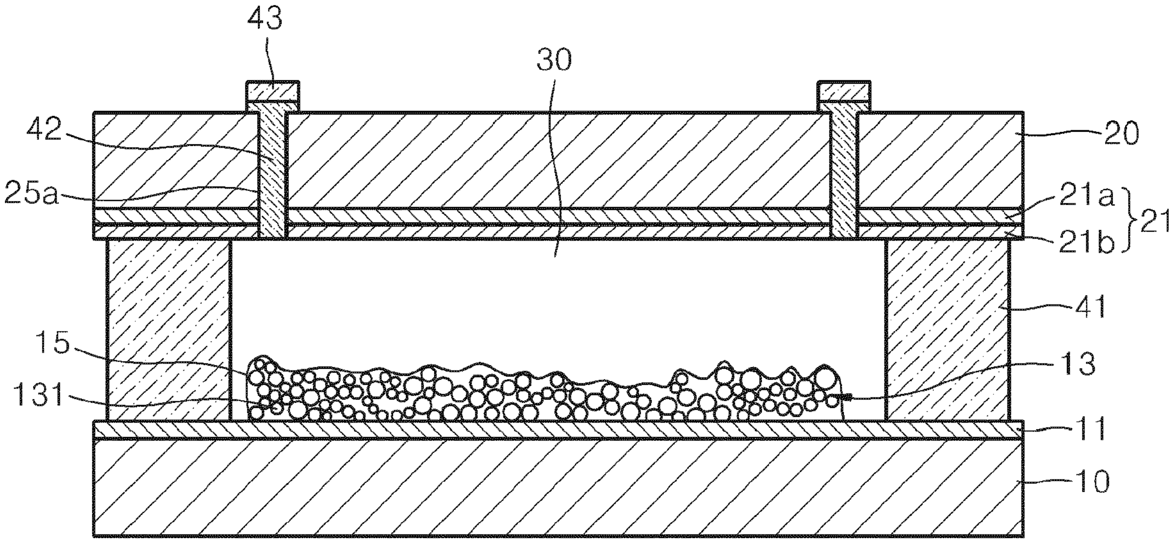

## Description

Any and all priority claims identified in the Application Data Sheet, or any correction thereto, are hereby incorporated by reference under 37 CFR 1.57. For example, this application claims the benefit of Korean Patent Application No. 10-2013-0025744, filed on March 11, 2013, in the Korean Intellectual Property Office, the disclosure of which is incorporated herein in its entirety by reference.

This disclosure relates to an electrolyte for a dye-sensitized solar cell and a dye-sensitized solar cell using the same.

A dye-sensitized solar cell typically includes a dye molecule-adsorbed semiconductor photocathode, an electrolyte including a redox ion couple, and a counter electrode including a platinum catalyst.

During operation a dye-sensitized solar cell receives light causing electrons to migrate from dye molecules to a semiconductor layer and ultimately to a counter electrode. Then, during the cycle the oxidized redox couple is reduced again by a catalyst coated on the surface of the counter electrode. The reduced redox couple reduces the oxidized dye to make a state ready for excitation. In this case, the potential difference between the conduction band of the semiconductor layer and the redox couple illustrates an open circuit voltage (Voc).

In the dye-sensitized solar cell, an electrolyte receives electrons from the counter electrode and functions to transfer the electrons to the dye molecule of the photocathode in the ground state. The electrolyte includes a redox couple for a reversible oxidation-reduction reaction, and a solvent dissolving the redox couple. The physical and chemical properties of the electrolyte are main factors determining the durability of the dye-sensitized solar cell.

The redox couple of the electrolyte is an essential material for transferring electrons and a core factor determining the overall performance of the solar cell , and typically includes an iodine-based material ($I^-/I_3$).

The conversion efficiency and the durability of the solar cell using the iodine-based material may not reach a satisfactory degree due to electrode corrosion by iodine when an iodine-based material is used as a redox couple.

According to a first aspect of the invention is provided an electrolyte for a dye-sensitized solar cell, which may not corrode an electrode, and a dye-sensitized solar cell including the same to have an improved durability and efficiency. According to one or more embodiments, an electrolyte for a dye-sensitized solar cell includes a redox couple including a compound represented by the following Formula 1,

**Formula 1**

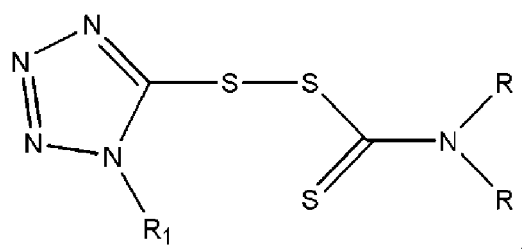

.

In Formula 1, R and $\mathbf{R_1}$ independently represent one group selected from a substituted or unsubstituted C1-C20 alkyl group, a substituted or unsubstituted C6-C20 aryl group, a substituted or unsubstituted C1-C20 heteroaryl group, a substituted or unsubstituted C4-C20 carbocyclic group, and a substituted or unsubstituted C1-C20 heterocyclic group.

According to a second aspect of the invention is provided a dye-sensitized solar cell includes a first electrode, a light absorbing layer formed on one surface of the first electrode, a second electrode disposed facing the first electrode including the light absorbing layer, and an electrolyte disposed between the first electrode and the second electrode.

According to the solar cell including the electrolyte for a dye-sensitized solar cell, the reliability due to the corrosion of the electrode may be enhanced and a solar cell having an improved efficiency may be provided.

These and/or other aspects will become apparent and more readily appreciated from the following description of the embodiments, taken in conjunction with the accompanying drawing in which:

FIG. 1 is a diagram illustrating the schematic structure of a dye-sensitized solar cell in accordance with an aspect of the present embodiments.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

Some embodiments provide an electrolyte for a dye-sensitized solar cell, including a redox couple including the compound represented by the following Formula 1,

**Formula 1**

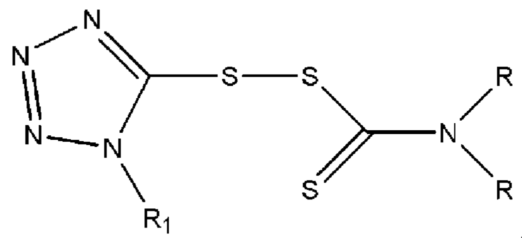

.

In Formula 1, **R** and **$R_1$** independently represent one group selected from a substituted or unsubstituted C1-C20 alkyl group, a substituted or unsubstituted C6-C20 aryl group, a substituted or unsubstituted C1-C20 heteroaryl group, a substituted or unsubstituted C4-C20 carbocyclic group, and a substituted or unsubstituted C1-C20 heterocyclic group.

In some embodiments of Formula 1, **R** and **$R_1$** may each independently be a C1-C10 alkyl group, for example, methyl, ethyl, or propyl.

In the invention, each R may be the same or each R may be different.

In some embodiments, the compound represented by Formula 1 may be a reaction product of a thiolate anion represented by the following Formula 3 and disulfide represented by the following Formula 4, wherein R and R1 are as defined above.

**Formula 3**

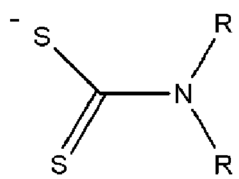

**Formula 4**

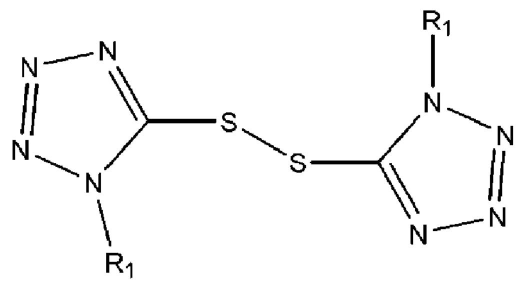

.

In some embodiments, the compound of Formula 1 is obtainable by reacting a thiolate anion represented by Formula 3 and a disulfide represented by Formula 4, wherein the thiolate anion represented by Formula 3 and the disulfide represented by Formula 4 are reacted in a molar ratio (Formula 3: Formula 4) of from about 2:1 to about 1: 2, or from about 1:1 to about 1:2.

In some embodiments, the thiolate anion represented by Formula 3 may be, for example, a compound represented by the following Formula 5.

## Formula 5

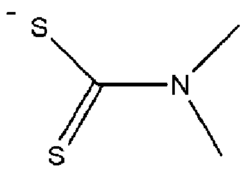

In some embodiments, the disulfide represented by Formula 4 may be, for example, a compound represented by the following Formula 6,

## Formula 6

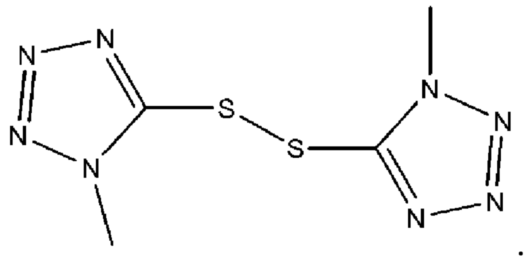

In some embodiments, the compound represented by Formula 1 may be, for example, a compound represented by the following Formula 2,

## Formula 2

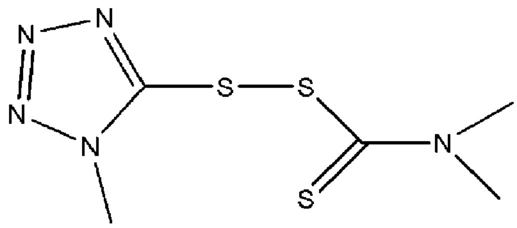

Referring to Scheme 1, reaction of a thiolate anion (M-) of Formula 5, and a disulfide **(T2)** of Formula 6 afford a compound (MT) represented by Formula 2.

## Scheme 1

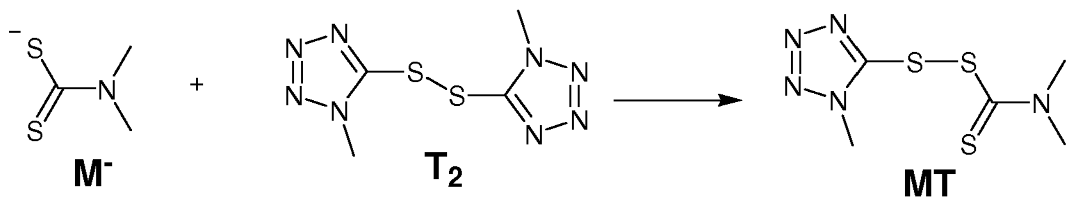

**M⁻** **$T_2$** **MT**

Referring to Reaction 1, the preparation process of the compound of Formula 2 may be understood.
In some embodiments, the electrolyte may include an organic solvent having a boiling point that is greater than or equal to 150°C.
In some embodiments, the organic solvent may be at least one selected from the group consisting of 3-methoxypropionitrile, N-methyl pyrrolidone (NMP), 1-methyl-3-methylimidazolium tetracyanoborate, 1-ethyl-3-methylimidazolium dicyanamide, 1-butyl-3-methylimidazolium dicyanamide, 1-butyl-3-methylimidazolium bis(trifluoromethylsulfonyl)imide, γ-butyrolactone (GBL), N-methyl-2-pyrrolidone (NMP), benzonitrile (BN), dimethyl sulfoxide (DMSO), N,N-dimethyl acetamide (DMAA), N,N-dimethyl ethaneamide (DMEA), 3-methoxypropionitrile (MPN), diglyme, N,N-diethyl formamide (DEF), and N,N-dimethyl formamide (DMF).
In some embodiments, the amount of the organic solvent is from about 100 to about 2,000 parts by weight

based on 100 parts by weight of the compound of Formula 1. When the amount of the organic solvent is in the range, stability may be good without deteriorating the efficiency of the electrolyte.

In some embodiments, the electrolyte may further include an additive to improve the current and voltage properties of a solar cell.

In some embodiments, the amount of the additive is from about 100 to about 1,500 parts by weight based on 100 parts by weight of the compound of Formula 1. When the amount of the additive is in the range, an electrolyte having good stability and good efficiency may be obtained.

Examples of the additive includes at least one selected from lithium perchlorate, tetrabutylammonium perchlorate, tetrabutylammonium hexafluorophosphate, tetrabutylammonium tetrafluoroborate, 1-ethyl-3-methylimidazolium tetracyanoborate, 4-tert-butylpyridine (TBP), 4-butylpyridine, pyrazole, imidazole, 1,2,3-triazole, 1,2,4-triazole, tetrazole, pyridazine, pyrimidine, pyrazine, 1,3,5-triazine, 2-aminoquinoline, 3-aminoquinoline, 5-aminoquinoline, and 6-aminoquinoline.

In some embodiments, the additive includes, for example, lithium perchlorate ($LiClO_4$) and TBP. By using the additive, an electrolyte having improved efficiency and stability may be prepared.

Since the electrolyte has good ion conductivity and includes minimized iodine amount, the efficiency and the stability thereof may be confirmed.

FIG. 1 is a cross-sectional view of a dye-sensitized solar cell in accordance with an aspect of the present embodiments.

Referring to FIG. 1, the dye-sensitized solar cell in accordance with an aspect of the present embodiments may include a first substrate **10** on which a first electrode **11** and a dye-sensitized photocathode **13** containing a dye **15** are formed, a second substrate **20** on which a second electrode **21** is formed and facing the first substrate **10,** and an electrolyte **30** disposed between the first electrode **11** and the second electrode **21**. A container (not illustrated) may be provided at the exterior of the first substrate **10** and the second substrate **20**. The structure will be described in more detail below.

In some embodiments, the first substrate **10** supporting the first electrode **11** may be transparent so that external light may transmit through it. In some embodiments, the first substrate **10** may be formed by using glass or plastic. The plastic may include, for example, polyethylene terephthalate (PET), polyethylene naphthalate (PEN), polycarbonate (PC), polypropylene (PP), polyimide (PI), triacetyl cellulose (TAC), and the like. In some embodiments, the transparency on visible light of the first substrate **10** may be greater than or equal to about 91%. In some embodiments, the thickness of the first substrate **10** may be 1 to 5 mm.

In some embodiments, the first electrode **11** formed on one surface of the first substrate **10** may be formed by using a transparent material such as at least one selected from indium tin oxide, indium oxide, tin oxide, zinc oxide, sulfur oxide, and fluorine oxide, $ZnO\text{-}Ga_2O_3$, or $ZnO\text{-}Al_2O_3$. In some embodiments, the first electrode 11 may be a single layer or an integrated layer of the transparent material.

In some embodiments, the dye-sensitized photocathode **13** may be formed on the first electrode **11**. In some embodiments, the dye-sensitized photocathode **13** may include titanium dioxide nanoparticles **131**. In some embodiments, the thickness of the dye-sensitized photocathode **13** may be from about 50,000 nm to about 20,000 nm. However, the thickness is not limited to the range, however, may vary according to the usage and technical development, etc.

For example, the photocathode may be formed by the following process. First, the titanium dioxide nanoparticles (oxide semiconductor particles), an acid, a binder and a solvent are mixed to prepare a composition for forming an oxide semiconductor layer (photocathode).

In some embodiments, the acid may include hydrochloric acid, nitric acid, acetic acid, and the like, and the amount of the acid may be from about 50 to about 300 parts by weight based on 100 parts by weight of the titanium dioxide nanoparticles. When the amount of the acid is in the above-described range, photocurrent characteristics of the photocathode manufactured from the oxide semiconductor layer may be good.

In some embodiments, the binder may include ethyl cellulose, hydropropyl cellulose, etc. and the amount of the polymer for the binder may be from about 5 to about 50 parts by weight based on 100 parts by weight of the titanium dioxide nanoparticles. When the amount of the binder is in this range, the photocurrent properties of the photocathode manufactured from the oxide semiconductor layer may be good.

In some embodiments, the solvent may include terpineol, ethanol, distilled water, ethylene glycol, alpha-terpineol, etc. and the amount of the solvent may be from about 200 to about 900 parts by weight based on 100 parts by weight of the titanium dioxide. When the amount of the solvent is in this range, the photocurrent properties of the photocathode manufactured from the oxide semiconductor layer may be good.

In some embodiments, the composition for forming the oxide semiconductor layer is coated on a substrate, that is, on the first electrode of the first substrate and then, is heat treated at from about 400°C to about 550°C to form the oxide semiconductor layer.

In some embodiments, the method for coating the composition for forming the oxide semiconductor layer on the substrate may include a spin coating method, a dip coating method, a casting method, a screen printing method,

and the like. In some embodiments, the thickness of the oxide semiconductor layer may be from about 1,000 to about 20,000 nm. In some embodiments, the coating and the heat treatment may be repeated for several times.

On the surface of the dye-sensitized photocathode **13,** the dye **15** for absorbing an external light to generate excited electrons is adsorbed.

In some embodiments, the dye **15** may be a dye including at least one metal selected from the group consisting of Al, Pt, Pd, Eu, Pb, Ir and Ru. For example, the dye **15** may be a ruthenium-based dye, however, may not be limited to these compounds but may be any dyes sensitizing to the solar light used in this technical field.

For example, the compounds have a good molar extinction coefficient and an improved photoelectric efficiency in a visible light region. In addition, the manufacturing unit cost of the compound is low, and the compound is an organic dye replaceable with the expensive inorganic dye, the ruthenium dye.

In some embodiments, the photosensitive dye may include the ruthenium-based dye $N_3$, and N719, Black Dye, and the like. The dye $N_3$ is $RuLS_2(NCS)_2$ (L=2,2'-bipyridyl-4,4'-dicarboxylic acid), and N719 represents $RuL_2(NCS)_2$:2 TBA (L=2,2'-bipyridyl-4,4'-dicarboxylic acid, TBA=tetra-n-butylammonium).

In some embodiments, a solution having a concentration of the photosensitive dye of from about 0.1 to about 7 mM may be prepared, and the photocathode may be dipped into the solution to adsorb the dye on the surface thereof. The concentration of the dye may be in any range only when the adsorption of the dye may be possible. In some embodiments, the solvent used may include ethanol, isopropanol, acetonitrile, and valeronitrile. However, the solvent may not be limited to these compounds but any solvents used in this technical art may be included.

In some embodiments, the second substrate **20** disposed so as to have a surface facing the first substrate **10**, functions as a supporter to support the second electrode **21** and may be transparent. In some embodiments, the second substrate **20** may be formed by using the same glass or plastic as the first substrate **10**.

In some embodiments, the second electrode **21** formed on the second substrate **20** may have a surface disposed to face the first electrode **11**. In some embodiments, the second electrode **21** may include a transparent electrode **21a** and a catalyst electrode **21b**. For example, the thickness of the transparent electrode **21a** may be from about 100 to about 1,000 nm. For example, the thickness of the catalyst electrode **21b** may be from about 1 to about 100 nm.

In some embodiments, the transparent electrode **21a** may include a transparent material such as indium tin oxide, fluoro tin oxide, antimony tin oxide, zinc oxide, tin oxide, $ZnO\text{-}Ga_2O_3$, $ZnO\text{-}Al_2O_3$, and the like. In some embodiments, the transparent electrode **21a** may be a single layer or an integrated layer of the transparent materials. In some embodiments, the catalyst electrode **21b** functions to activate a redox couple and includes platinum, ruthenium, palladium, iridium, rhodium (Rh), osmium (Os), carbon (C), $WO_3$, $TiO_2$, and the like.

In some embodiments, the first substrate **10** and the second substrate **20** may be connected by an adhesive **41**, and the electrolyte **30** according to an exemplary embodiment may be injected between the first electrode **11** and the second electrode **21** through a hole **25a** penetrating the second substrate **20** and the second electrode **21.** In some embodiments, the electrolyte **30** may be uniformly dispersed into the dye-sensitized photocathode **13.** In some embodiments, the electrolyte **30** receives electrons from the second electrode **21** by an oxidation and reduction and transfers the electrons to the dye **15.** In some embodiments, the hole **25a** penetrating the second substrate **20** and the second electrode **21** may be sealed by an adhesive **42** and a cover glass **43.**

Even though not illustrated in FIG. 1, on the first electrode **11** and under the dye-sensitized photocathode **13**, a metal oxide layer as a porous layer may be additionally formed.

In some embodiments, the porous layer may be formed by using metal oxide particles and may include titanium oxide, zinc oxide, tin oxide, strontium oxide, indium oxide, iridium oxide, lanthanum oxide, vanadium oxide, molybdenum oxide, tungsten oxide, niobium oxide, magnesium oxide, aluminum oxide, yttrium oxide, scandium oxide, samarium oxide, gallium oxide, strontium titanium oxide, and the like. Here, according to example embodiments of the metal oxide particles, titanium dioxide ($TiO_2$), tin oxide ($SnO_2$), tungsten oxide ($WO_3$), zinc oxide (ZnO), or a complex thereof may be used. Hereinafter, the definition on the substituent used in the chemical formulae will be described.

As use herein, "alkyl" refers to a linear type or a branched hydrocarbon group that is fully saturated (i.e., contains no double or triple bonds). Typical alkyl groups include, but are in no way limited to, methyl, ethyl, propyl, isobutyl, sec-butyl, pentyl, iso-amyl, hexyl, heptyl, octyl, nonanyl, dodecyl, and the like. In some embodiments, at least one hydrogen atom in the alkyl group may be substituted with a deuterium atom, a halogen atom, a hydroxyl group, a nitro group, a cyano group, an amino group, an amidino group, hydrazine, hydrazone, a carboxyl group or a salt thereof, a sulfonic acid group or a salt thereof, phosphoric acid or a salt thereof, a $C_1$-$C_{10}$ alkoxy group, a $C_2$-$C_{10}$ alkenyl group, a $C_2$-$C_{10}$ alkynyl group, a $C_6$-$C_{16}$ aryl group, or a $C_4$-$C_{16}$ heteroaryl group.

As use herein, "aryl" refers to an aromatic ring or ring system (i.e., two or more fused rings that share two adjacent carbon atoms) containing only carbon in the ring backbone. Examples of aryl groups include, but are not limited to, phenyl, naphthyl, and tetrahydronaphtyl. In some embodiments, the aryl group may be substituted by a substituent as listed for the alkyl group.

As use herein, "heteroaryl" refers to refers to an aromatic ring or ring system (i.e., two or more fused rings that share two adjacent atoms) that contain(s) one or more heteroatoms, that is, an element other than carbon, including but

not limited to, N (nitrogen), O (oxygen), P (phosphorus), and S (sulfur). At least one hydrogen atom in the heteroaryl group also may be substituted by the same substituent as listed for the alkyl group.

As use herein, "heterocyclic" refers to a cyclic ring or ring system including a heteroatom such as nitrogen, sulfur, phosphorus, oxygen, etc., and at least one hydrogen atom in the heterocyclic group may be substituted by a substituent as listed for the alkyl group.

As use herein, "carbocyclic" refers to a non-aromatic cyclic ring or ring system containing only carbon atoms in the ring system backbone. At least one hydrogen atom in the carbocyclic group may be substituted by the substituent as for the alkyl group. Hereinafter, the present invention will be described in more detail referring to example embodiments. Example embodiments are illustrated only for explaining the present invention however, the scope of the present invention is not limited to the embodiments.

EXAMPLES

**Preparation Example 1: Preparation of electrolyte**

Di-5-(1-methyltetrazole) disulfide ($T_2$) of Formula 6 was dissolved in γ-butyrolactone to prepare a γ-butyrolactone solution of 0.1M di-5-(1-methyl tetrazole) disulfide ($T_2$). Separately, sodium dimethyldithiocarbamate hydrate ($Na^+M^-$) was dissolved in gamma butyrolactone to prepare a γ-butyrolactone solution of 0.1M sodium dimethyldithiocarbamate hydrate.

Subsequently, 3 mL of the γ-butyrolactone solution of 0.1M di-5-(1-methyltetrazole) disulfide (T2) and 3 mL of the γ-butyrolactone solution of 0.1M sodium dimethyldithiocarbamate hydrate were mixed with 0.0213 g of a γ-butyrolactone solution of 0.5M 4-tert-butylpyridine (TBP) to prepare a 0.4M electrolyte (in γ-butyrolactone) including the compound of Formula 2.

**Preparation Examples 2 and 3: Preparation of electrolyte**

The same procedure as described in Preparation Example 1 was conducted except for changing the molar concentrations of $T_2$ and M- as illustrated in Table 1 for samples 2 and 3 to prepare electrolytes.

**Table 1**

| Sample | Concentration of [M-] (M) | Concentration of [$T_2$] (M) |
|---|---|---|
| 1 | 0.1 | 0.1 |
| 2 | 0.1 | 0.2 |
| 3 | 0.1 | 0.4 |

**Comparative Preparation Example 1: Preparation of electrolyte (T-/$T_2$)**

Di-5-(1-methyltetrazole) disulfide ($T_2$) was dissolved in gamma butyrolactone to prepare a γ-butyrolactone solution of 0.1M di-5-(1-methyltetrazole) disulfide ($T_2$). 5-Mercapto-1-methyltetrazole N-tetramethyl ammonium ($^+NMe_4T^-$) was dissolved in γ-butyrolactone to prepare a γ-butyrolactone solution of 0.1M 5-mercapto-1-methyltetrazole N-tetramethyl ammonium ($^+NMe_4T^-$).

Subsequently, 3 mL of the γ-butyrolactone solution of 0.1M di-5-(1-methyltetrazole) disulfide ($T_2$) and 3 mL of the γ-butyrolactone solution of 0.1M 5-mercapto-1-methyltetrazole N-tetramethyl ammonium ($^+NMe_4T^-$) were mixed with 0.0213 g of a y-butyrolactone solution of 0.5M TBP to prepare a 0.4M electrolyte (in γ-butyrolactone).

**Comparative Preparation Example 2: Preparation of electrolyte (T-/$T_2$)**

Tetramethylthiuram disulfide ($M_2$) was dissolved in γ-butyrolactone to prepare a γ-butyrolactone solution of 0.1M tetramethylthiuram disulfide.

5-Mercapto-1-methyltetrazole N-tetramethyl ammonium ($^+NMe_4T^-$) was dissolved in γ-butyrolactone to prepare a γ-butyrolactone solution of 0.1M 5-mercapto-1-methyltetrazole N-tetramethyl ammonium ($^+NMe_4T^-$).

Subsequently, 3 mL of the γ-butyrolactone solution of 0.1M tetramethylthiuram disulfide and 3 mL of the γ-butyrolactone solution of 0.1M 5-mercapto-1-methyltetrazole N-tetramethyl ammonium were mixed with 0.0213 g of γ-butyrolactone solution of 0.5M TBP to prepare a 0.4M electrolyte (in γ-butyrolactone).

**Comparative Preparation Example 3: Preparation of electrolyte ($M^-/M_2$)**

Tetramethylthiuram disulfide ($M_2$) was dissolved in γ-butyrolactone to prepare a γ-butyrolactone solution of 0.1M tetramethylthiuram disulfide.

Sodium dimethyldithiocarbamate hydrate (Na+M-) was dissolved in γ-butyrolactone to prepare a γ-butyrolactone solution of 0.1M sodium dimethyldithiocarbamate hydrate. Subsequently, 3 mL of the γ-butyrolactone solution of 0.1M tetramethylthiuram disulfide and 3 mL of the γ-butyrolactone solution of 0.1M sodium dimethyldithiocarbamate hydrate were mixed with 0.0213 g of TBP to prepare a 0.4M electrolyte (in γ-butyrolactone).

**Example 1: Manufacture of dye-sensitized solar cell**

On a fluorine-doped tin oxide (FTO) substrate (thickness: 2.3 mm), a $TiO_2$ paste (PST-18NR, JGC C&C, Kawasaki-shi Japan) was coated to a thickness of about 12 μm by screen printing and baked at a temperature increasing rate of 10°C/min at 500°C for 30 minutes. Then, a scattering particle paste (400c, JGC C&C, Kawasaki-shi Japan) was printed/baked by the same method. After baking, a photocathode having a thickness of about 4 μm was manufactured.

The manufactured photocathode was dipped into a dye solution (0.2 mM N719/EtOH) and allowed to stand for 24 hours.

Separately, a carbon black paste was coated and dried at 120°C for 20 minutes to manufacture a carbon electrode used as a counter electrode.

A hot melt film (60 μm, Suryln, DuPont, Wilmington, Delaware) was inserted into the photocathode and the counter electrode including holes and heat sealed (130°C/15 sec) by using a hot press. The electrolyte prepared according to Preparation Example 1 was injected through the holes formed in the counter electrode to manufacture a dye-sensitized solar cell.

**Examples 2 and 3: Manufacture of dye-sensitized solar cell**

A dye-sensitized solar cell was manufactured by conducting the same procedure described in Example 1 except for using the electrolytes according to Preparation Examples 2 and 3 instead of the electrolyte according to Preparation Example 1.

**Comparative Example 1: Manufacture of dye-sensitized solar cell**

A dye-sensitized solar cell was manufactured by conducting the same procedure described in Example 1 except for using the electrolyte according to Comparative Preparation Example 1 instead of the electrolyte according to Preparation Example 1.

**Comparative Example 2: Manufacture of dye-sensitized solar cell**

A dye-sensitized solar cell was manufactured by conducting the same procedure described in Example 1 except for using the electrolyte according to Comparative Preparation Example 2 instead of the electrolyte according to Preparation Example 1.

**Comparative Example 3: Manufacture of dye-sensitized solar cell**

A dye-sensitized solar cell was manufactured by conducting the same procedure described in Example 1 except for using the electrolyte according to Comparative Preparation Example 3 instead of the electrolyte according to Preparation Example 1.

**Evaluation Example 1: Evaluation on performance of dye-sensitized solar cell**

**Performance of dye-sensitized solar cell according to Examples 1 to 3 and Comparative Examples 1 to 3**

In the dye-sensitized solar cells manufactured according to Examples 1 to 3 and Comparative Examples 1 to 3, current-voltage curves under standard measuring conditions (AM1, 5G, 100 $mWcm^{-2}$) were evaluated. In addition, the measuring conditions on an open circuit voltage, a photocurrent density, an energy conversion efficiency (eff), and a fill factor (FF) are as follows:

(1) Open circuit voltage (Voc) and photocurrent density (Jsc)

The open circuit voltage and the photocurrent density were measured by using a Source Measure Unit (SMU) Instrument (SMU2400, Keithley Instruments, Inc., Cleveland, Ohio).

(2) Energy conversion efficiency (eff) and FF

The measure on the eff was conducted by using 1.5AM 100 mW/cm$^2$ solar simulator (including Xe lamp [300 W, Oriel Corporation, Stratford, CT], AM1.5 filter, and Keithley SMU2400), and the FF was calculated by using the conversion efficiency and the following Calculating Equation:

**Calculating Equation**

$$Fill\ Factor(\%) = \frac{(J \times V)\max}{Jsc \times Voc} \times 100$$

In the Calculating Equation, J is a Y-axis value in a conversion efficiency curve, V is an X-axis value in a conversion efficiency curve, and Jsc is an intercept of each axis. Dye-sensitized solar cells according to Examples 1 to 3 and Comparative Example 1 were manufactured by using the electrolytes according to Preparation Example 1 and Comparative Preparation Example 1. Current-voltage properties on the dye-sensitized solar cells were analyzed by using a 100 mW/cm$^2$ xenon lamp as a light source, and the results are illustrated in the following Table 2:

**Table 2**

| **Division** | **Initial efficiency** | | | |
|---|---|---|---|---|
| | **Voc (V)** | **Jsc (mA cm$^2$j** | **FF(%)** | **eff. (%)** |
| Comparative Example 1 | 0.646 | 6.4 | 18.8 | 0.78 |
| Comparative Example 2 | 0.626 | 6 | 30.3 | 1.3 |
| Comparative Example 3 | 0.616 | 4.7 | 45 | 1.3 |
| Example 1 | 0.590 | 8.7 | 58 | 3.1 |
| Example 2 | 0.580 | 9.5 | 52 | 2.9 |
| Example 3 | 0.390 | 6.8 | 50 | 0.9 |

From Table 2, the performance of the dye-sensitized solar cells such as the open circuit voltage, the photocurrent density, the eff and the FF according to Examples 1 to 3 is improved when compared with that of Comparative Examples 1 to 3. In particular, the photocurrent density of Examples 1 to 3 ranges from 6.8 to 9.8 mA cm$^{-2}$. In contrast, the photocurrent density of Comparative Examples 1 to 3 ranges from 4.7 to 6.4 mA cm$^{-2}$. Further, the fill factor of Examples 1 to 3 ranges from 50% to 58%. In contrast, the fill factor of Comparative Examples 1 to 3 ranges from 18.8% to 45%.

In the present disclosure, the terms "Example," "Comparative Example," "Preparation Example," "Comparative Preparation Example," and "Evaluation Example" are used arbitrarily to simply identify a particular example or experimentation and should not be interpreted as admission of prior art. It should be understood that the exemplary embodiments described therein should be considered in a descriptive sense only and not for purposes of limitation. Descriptions of features or aspects within each embodiment should typically be considered as available for other similar features or aspects in other embodiments.

## Claims

**1.** An electrolyte for a dye-sensitized solar cell comprising a redox couple including a compound represented by Formula 1,

**Formula 1**

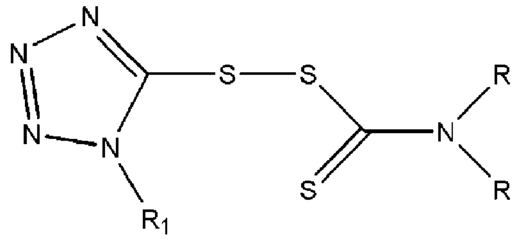

wherein, R and $\mathbf{R_1}$ each independently are selected from a substituted or unsubstituted C1-C20 alkyl group, a substituted or unsubstituted C6-C20 aryl group, a substituted or unsubstituted C1-C20 heteroaryl group, a substituted or unsubstituted C4-C20 carbocyclic group, and a substituted or unsubstituted C1-C20 heterocyclic group.

2. The electrolyte for a dye-sensitized solar cell of claim 1, wherein R and $\mathbf{R_1}$ are each independently a substituted or unsubstituted C1-C10 alkyl group.

3. The electrolyte for a dye-sensitized solar cell of claim 1, wherein R and $\mathbf{R_1}$ are each independently methyl, ethyl, or propyl.

4. The electrolyte for a dye-sensitized solar cell of claim 1, wherein the compound represented by Formula 1 is a compound represented by Formula 2,

**Formula 2**

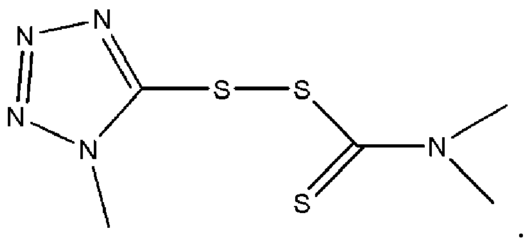

5. The electrolyte of any one of the preceding claims, wherein the compound of Formula 1 is obtainable by reacting a thiolate anion represented by Formula 3 and a disulfide represented by Formula 4,

**Formula 3**

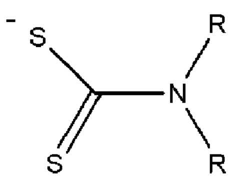

**Formula 4**

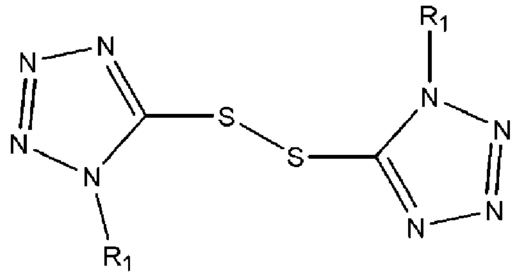

wherein R and R1 are as defined in Formula 1; and
wherein the thiolate anion represented by Formula 3 and the disulfide represented by Formula 4 are reacted in a molar ratio (Formula 3: Formula 4) of from about 2:1 to about 1: 2, or from about 1:1 to about 1:2.

**6.** The electrolyte for a dye-sensitized solar cell of any one of the preceding claims, further comprising an organic solvent.

**7.** The electrolyte for a dye-sensitized solar cell of claim 6, wherein a boiling point of the organic solvent is greater than or equal to about 150°C.

**8.** The electrolyte for a dye-sensitized solar cell of claim 6, wherein the organic solvent is at least one selected from the group consisting of 3-methoxypropionitrile, N-methyl pyrrolidone, 1-methyl-3-methylimidazolium tetracyanoborate, 1-ethyl-3-methylimidazolium dicyanamide, 1-butyl-3-methylimidazolium dicyanamide, 1-butyl-3-methylimidazolium bis(trifluoromethylsulfonyl)imide, γ-butyrolactone, benzonitrile, dimethyl sulfoxide, N,N-dimethyl acetamide, N,N-dimethyl ethaneamide, 3-methoxypropionitrile, diglyme, N,N-diethyl formamide, and N,N-dimethyl formamide.

**9.** The electrolyte for a dye-sensitized solar cell of any one of claims 6 to 8, wherein an amount of the organic solvent is from about 100 to about 2,000 parts by weight based on 100 parts by weight of the compound of Formula 1.

**10.** The electrolyte for a dye-sensitized solar cell of any one of the preceding claims, further comprising an additive that is at least one selected from the group consisting of lithium perchlorate, tetrabutylammonium perchlorate, tetrabutylammonium hexafluorophosphate, tetrabutylammonium tetrafluoroborate, 1-ethyl-3-methylimidazolium tetracyanoborate, 4-tert-butylpyridine, 4-butylpyridine, pyrazole, imidazole, 1,2,3-triazole, 1,2,4-triazole, tetrazole, pyridazine, pyrimidine, pyrazine, 1,3,5-triazine, 2-aminoquinoline, 3-aminoquinoline, 5-aminoquinoline, 6-aminoquinoline, and mixtures thereof.

**11.** The electrolyte for a dye-sensitized solar cell of claim 10, further comprising lithium perchloroate and 4-tert-butylpyridine.

**12.** The electrolyte for a dye-sensitized solar cell of claim 10, wherein an amount of the additive is from about 100 to about 1,500 parts by weight based on 100 parts by weight of the compound of Formula 1.

**13.** A dye-sensitized solar cell comprising:

a first electrode;
a light absorbing layer formed on one surface of the first electrode;
a second electrode facing the first electrode and on which the light absorbing layer is formed; and
an electrolyte according to any one of the preceding claims, wherein the electrolyte is between the first electrode and the second electrode.

FIG. 1

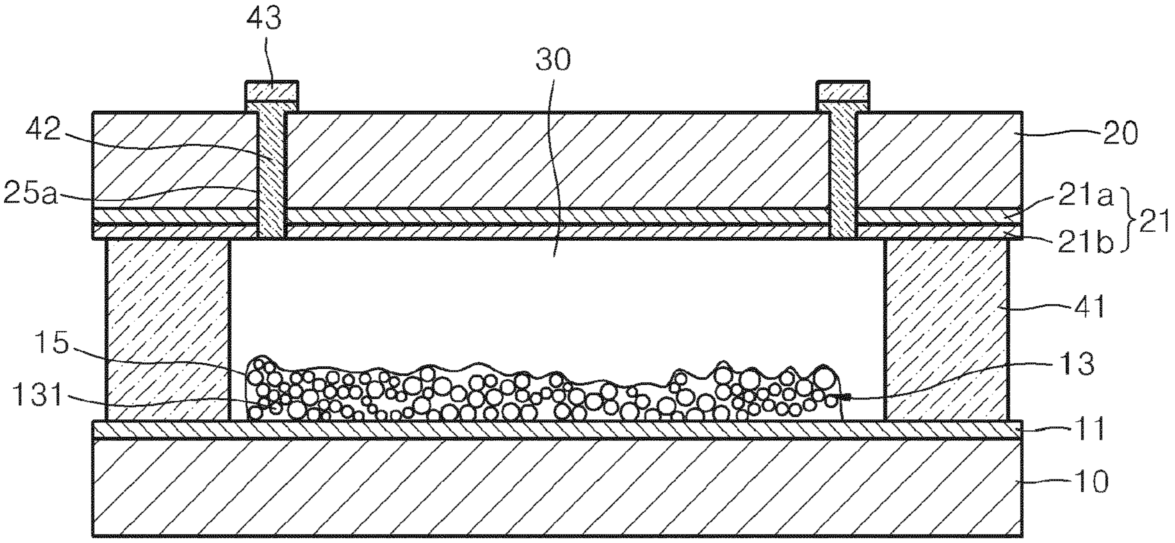

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020130025744